# EUROPEAN PATENT SPECIFICATION

(11) **EP 1 751 551 B1**
(45) Date of publication and mention of the grant of the patent: **16.12.2009**
(21) Application number: 05743191.8
(22) Date of filing: 25.05.2005
(51) Int. Cl.: G01N 33/543, G01N 33/539

(54) **PROCESS FOR DETERMINING BINDING PARAMETERS OF BIOAFFINITY BINDING REACTIONS**
VERFAHREN ZUR BESTIMMUNG DER BINDUNGSPARAMETER VON BIOAFFINITÄTSBINDUNGSREAKTIONEN
PROCEDE PERMETTANT DE DETERMINER DES PARAMETRES DE LIAISON DE REACTIONS DE LIAISON DE BIOAFFINITE

(30) Priority: 01.06.2004 EP 04076608
(43) Date of publication of application: 14.02.2007
(73) Proprietor: Nederlandse Organisatie voor Toegepast-Natuurwetenschappelijk Onderzoek TNO, 2628 VK Delft (NL)
(72) Inventor: HERMENS, Willem, Theodoor, NL-6247 CV Gronsveld (NL); SPEIJER, Johan, Gerhard, NL-6367 BJ Voerendaal (NL)
(74) Representative: van Loon, C.J.J.
(86) International application number: PCT/EP2005/005752
(87) International publication number: WO 2005/119257

(56) References cited:
- EP-A- 0 919 811
- WO-A-03/056296
- ROBERS MARKUS ET AL: "A new principle for rapid immunoassay of proteins based on in situ precipitate-enhanced ellipsometry" BIOPHYSICAL JOURNAL, vol. 76, no. 5, May 1999 (1999-05), pages 2769-2776, XP002293663 ISSN: 0006-3495
- SPEIJER H ET AL: "One-step immunoassay for measuring protein concentrations in plasma, based on precipitate-enhanced ellipsometry" ANALYTICAL BIOCHEMISTRY, ACADEMIC PRESS, SAN DIEGO, CA, US, vol. 326, no. 2, 15 March 2004 (2004-03-15), pages 257-261, XP004493120 ISSN: 0003-2697
- NOORT VAN D ET AL: "SILICON BASED AFFINITY BIOCHIPS VIEWED WITH IMAGING ELLIPSOMETRY" MEASUREMENT SCIENCE AND TECHNOLOGY, IOP PUBLISHING, BRISTOL, GB, vol. 11, no. 6, June 2000 (2000-06), pages 801-808, XP001073134 ISSN: 0957-0233
- YOUNG MIN BAE ET AL: "IMMUNOSENSOR FOR DETECTION OF YERSINIA ENTEROCOLITICA BASED ON IMAGING ELLIPSOMETRY" ANALYTICAL CHEMISTRY, AMERICAN CHEMICAL SOCIETY. COLUMBUS, US, vol. 76, no. 6, 15 March 2004 (2004-03-15), pages 1799-1803, XP001196662 ISSN: 0003-2700
- FAEGERSTAM L G ET AL: "BIOSPECIFIC INTERACTION ANALYSIS USING SURFACE PLASMON RESONANCE DETECTION APPLIED TO KINETIC, BINDING SITE AND CONCENTRATION ANALYSIS" JOURNAL OF CHROMATOGRAPHY, ELSEVIER SCIENCE PUBLISHERS B.V. AMSTERDAM, NL, vol. 597, 1992, pages 397-410, XP000973892 ISSN: 0021-9673
- MAY LEE MAY ET AL: "Novel determination of cadmium ions using an enzyme self-assembled monolayer with surface plasmon resonance." ANALYTICA CHIMICA ACTA, vol. 500, no. 1-2, 19 December 2003 (2003-12-19), pages 119-125, XP002293664 ISSN: 0003-2670

## Description

The present invention relates to a process for determining binding parameters, including dissociation constants and sorption rate constants, of the binding between a biomolecule and a binding partner thereof, both being present in a liquid phase, and comprising a marker-free binding step.

Processes for determining binding parameters of the marker-free binding between biomolecules and their binding partners are generally known in the art. A relevant review is provided by Haake, H.M., Schütz, A. en Gauglitz, G., Fresenius J. Anal. Chem. (2000) 366:576-585.

As it is generally known in the state of the art, the dissociation constant (abbreviated as K_{d}) of a particular binding is defined as the ratio between the so-called "on-sorption rate constant" (abbreviated as kₒₙ) and the "off-sorption rate constant" or desorption rate constant (abbreviated as k_{off}), which can be expressed by the equation K_{d} = k_{off}/kₒₙ.

In the context of the present invention, the term "biomolecule" is defined as a molecule with a biological origin, such as, but not limited to, a protein, nucleic acid, lipid, carbohydrate, steroid and prostaglandin, which is formed in vivo or in vitro, and, in the latter case, is either identical or equivalent to the natural form of the biomolecule, which biomolecule usually, but not necessarily, has a biological or physiological activity.

In the context of the present invention, the term "binding partner" is defined as a molecule having an affinity, and to a certain degree also specificity, for a particular biomolecule. The binding partner usually, but not necessarily, is a low molecular weight organic compound (e.g. a steroid, where the biomolecule is a cell surface receptor protein; or an organic substrate molecule, where the biomolecule is an enzyme); however, it can itself also be a biomolecule (e.g. an antibody, which is essentially a protein, where the biomolecule is an antigen, which antigen can itself be a protein).

Essentially, the prior art methods (FÄGERSTAM et al. (1992), J. Chromatog., 597, 397-410; WO-A-03/056296) measure the binding of a biomolecule (such as an antibody) on solid surfaces coated with a binding partner for a biomolecule (such as an antigen) in buffer solutions. Usually, the surface is covered with e.g. the biomolecule, either by covalent coupling or by physical adsorption. The value of K_{d} is then estimated for a series of increasing concentrations of the binding partner, essentially as the concentration for which binding is half-maximal. Alternatively, the value for the rate constant of adsorption (kₒₙ) is estimated from the initial adsorption phase, and the value for the rate constant of desorption (k_{off}) is then estimated by removing the binding partner (e.g. by washing or flushing) and in some way measuring the desorption.

However, several disadvantages are associated with the prior art processes for determining binding parameters, and particularly so if the K_{d} values involved are lower than 10⁻⁹ M. Firstly, even for transport-limited adsorption of the binding partner (which is usually not attained), the adsorption of the binding partner will in practice take too much time when binding partner concentrations become as low as 10⁻¹⁰ M - 10⁻¹¹ M. Secondly, due to the high kₒₙ values involved, the renewed adsorption of binding partner during flushing or washing (which is required for each concentration of biomolecule or binding partner) is significant, and the so-called "apparent k_{off}" (abbreviated as k_{off},ₐₚₚ) becomes much lower than the value of the true k_{off}. Furthermore, weak and aspecific interactions of adsorbed binding partner molecules with the surface may undesirably interfere with desorption, even to the point that the binding becomes virtually irreversible.

There is a need for a process which is less, or preferably not, subject to the aforementioned limitations concerning measurements when the dissociation constants are lower than 10⁻⁹ M. Furthermore, there is a need for a process for determining binding parameters of the binding between a biomolecule and a binding partner thereof which comprises fewer steps, and is therefore less laborious and cumbersome than the prior art method.

The process for determining binding parameters according to the present invention is as claimed in claims 1-15 and is characterised by the following steps:
(a) fixating either (a1) the binding partner, or (a2) the biomolecule, to a solid surface;
(b) allowing binding between the binding partner and the biomolecule, both in the liquid phase and on the solid surface;
(c) attaching a marker to: (c1) the biomolecule, if said binding partner was fixated to the solid surface according to step (a1), or to (c2) the binding partner, if said biomolecule was fixated to the solid surface according to step (a2);
(d) allowing the marker to produce a precipitate; and
(e) detecting said precipitate as it is formed on said solid surface by determining a change of surface mass on the solid surface due to the formation of said precipitate.

The solid surface is used as a measuring tool when measuring the binding equilibrium in the liquid phase. The binding partner or the biomolecule is fixated to a pretreated solid surface (step a). The amount of binding to the surface by the binding partner (or, respectively, the biomolecule) is a measure for the free concentration of the binding partner (or, respectively, the biomolecule) in the liquid phase (step b). Amplification of the signal is necessary for measuring the often very low concentrations, which is achieved by attaching a marker to the biomolecule (or, respectively, to the binding partner) (step c). The binding as such is marker-free (thus avoiding marker-induced changes of binding affinities and a possible error in the estimation of the dissociation constant).The marker is then allowed to produce a precipitate (step d) and the velocity of the formation of said precipitate on the solid surface is detected by determining a change of surface mass on the solid surface due to the formation of said precipitate.

An advantage of the present process is that, as the precipitate is detected by determining a change of surface mass on the solid surface, only the substrate converted directly at the surface will contribute to precipitate formation. Small aggregates of converted substrate in the liquid phase will not interfere with the measurement. This actually leads to a so-called one-step assay process for determining binding parameters, without the cumbersome and laborious intervening washing or flushing steps which are obligatory when using prior art processes. According to the present invention, equilibrium is obtained fairly quickly. The free binding partner (or free biomolecule) concentration can be determined by comparing the rate of the formation of the precipitate with a standard curve. From the free concentrations thus measured, the binding constants are then calculated. A major advantage of the process according to the invention is that the free concentrations can be measured while in equilibrium with complexes of the biomolecule and the binding partner thereof. The above-mentioned aspecific interactions, as well as the problems involved with differences between true sorption constants and apparent sorption constants, which may cause considerable errors, are therefore avoided when using the process according to the invention. Finally, and as will be illustrated hereinafter, the surface area of the present solid surface according to the invention can be kept very small, thereby preventing any significant influence of protein adsorption on the free protein concentration.

A preferred embodiment of the present invention relates to a process for determining binding parameters, in which the biomolecule is an enzyme and the binding partner is an inhibitor or a substrate therefor. According to another preferred embodiment, the biomolecule is a cell membrane protein and the binding partner is ligand therefor. Preferably, said cell membrane protein is a receptor. According to another embodiment of the present invention, the biomolecule is a cell membrane protein and the binding partner is a cell membrane structure. Preferably, said cell membrane structure is artificial, and according to a particular embodiment, the cell membrane structure is a complete cell.

A preferred variant of the present invention relates to a process in which the biomolecule is an antibody or a fragment thereof, and the binding partner is an antigen of said antibody or fragment. Preferably, the marker of the present process is an enzyme producing a precipitate from a soluble substrate.

Particularly preferred embodiments of the present solid surface are (i) a silicon slide, and (ii) a chromium-sputtered glass.

Although step (e) of the present process can be carried out in several ways, it is preferred that the determination of the change of surface mass is carried out by ellipsometry. However for carrying out the method according to the invention other analysing techniques for determining of the change of surface mass can be implemented, for example an analysing technique based on the principle of Surface Plasmon Resonance (SPR) or Total Internal Reflection Fluorescence (TIRF). With both techniques (SPR and TIRF), but in particular with the SPR-technique similar results can be achieved.

Preferably, the step of determining a change of surface mass is carried out on the surface area of less than 3.0 mm².

According to a particular embodiment of the present process, the dissociation constant is lower than 10⁻⁹ M, and is in particular 10⁻⁹ M - 10⁻¹³ M.

Further disclosed is a slide for carrying out the present process. The slide should be covered with a protein-adsorbing layer, preferably a synthetic polymer. Preferably, the slide is cut from a phosphorous-doped and silica-coated silicon wafer, and is covered with a PVC layer or a polystyrene layer. It is preferred that the PVC layer has a thickness of 10-30 nm. According to another preferred embodiment of the aforementioned slide, it is covered with a silane layer, which preferably has a thickness of 1-5 nm. Slides can be modified in order to enable attachment of a biomolecule or binding partner. A preferred slide is characterised in that it is cut from a phosphorous-doped and silica-coated silicon wafer and is modified by attachment of a molecule with an amino group. The amino group is then available for covalent binding of the biomolecule or binding partner thereof, by a treatment with N-succinimidyl-3-(2-pyridyldithio)proprionate (SPDP).

Finally, also disclosed is a kit for carrying out the process according to the present invention, comprising at least a solid surface consisting of silicon slides or chromium sputtered glass, and either a marked binding partner or a marked biomolecule. Preferably, said kit also contains a standard of the binding partner or the biomolecule to be determined. Further disclosed is a kit for carrying out the process according to the invention, comprising at least a solid surface capable of immobilising the biomolecule or its binding partner, or with the biomolecule or its binding partner already bound. In a particular embodiment the biomolecule or its binding partner is a murine monoclonal antibody, and the marker is a HRP-labelled secondary antibody which binds to mouse monoclonal antibodies. The kit preferably comprises at least a solid surface consisting of silicon slides or chromium sputtered glass as well as a marker which binds to any antibody used as the binding partner of a biomolecule that was previously adsorbed on the slide. In particular embodiments, HRP-labelled Protein A or Protein G are used as a marker.

The present invention can be used to study different kinds of interactions, examples of which are (i) the interaction between a protein and a membrane (or membrane component), (ii) the interaction between an enzyme and an inhibitor thereof, (iii) the interaction between a membrane-bound receptor and a ligand for said receptor, and (iv) the interaction between an antibody and an antigen.

The invention will be illustrated by the following nonlimiting examples.
Figure 1 shows an example of the determination of the dissociation constant of an protein-membrane interaction;
Figure 2 shows an example of the determination of the dissociation constant of an enzyme-inhibitor interaction in the usual range of values;
Figure 3 shows the determination of the dissociation constant for the IL6-α126.16 complex in blood plasma, in the high affinity range of constants;
Figure 4a-4b show examples of the determination of binding constants of α-IL6.16.

### Example 1:

### Determination of the binding constants of a protein-membrane interaction

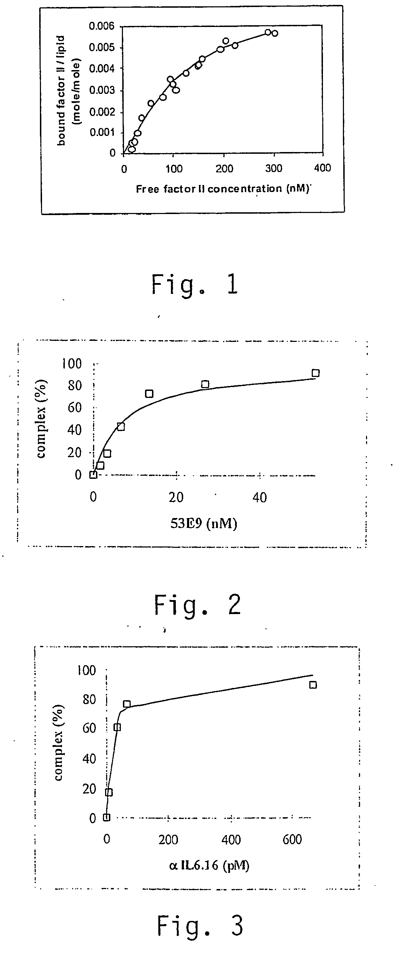

Small unilamellar vesicles (SUVs) were prepared by sonication of a phospholipid mixture of 20% dioleoyl-phosphatidylserine (DOPS) and 80% dioleoyl-phosphatidylcholine (DOPC). Various concentrations (25-500 nM) of purified bovine factor II were added to 0.05 mM (total phospholipid) SUVs in 0.05 M Tris-HCl buffer (pH 7.5), containing 0.1 M NaCl, 3 mM CaCl₂, 0.5 g/l bovine serum albumin, and 100 ng/ml of a rabbit anti-bovine factor II-HRP conjugate. Silica surfaces were covered with phospholipid bilayers by previous exposure of the slides to 0.020 mM SUVs. The free concentrations of factor II were measured by ellipsometry from the precipitation rates after addition of DAB-H₂O₂ substrate, using a commercially available ellipsometer (EL-X-02C, Dr. Riss Ellipsometerbau, GmbH, Ratzeburg, Germany). Results are shown in Figure 1 and a value of K_{d} = 123 ± 21 nM was obtained.

### Example 2:

### Determination of the dissociation constant for the FABP-53E9 complex in buffer

FABP was used as antigen and a monoclonal anti-FABP (53E9) was used as the corresponding antibody. Both were a kind gift of Prof. J. Glatz, Department of Physiology, CARIM. The dissociation constant K_{d} of this antibody has been reported to be 13,5 nM (Roos et al, J. Immunol. Meth. (1995) 183: 149-153). FABP (0,67 nM) was incubated during 30 minutes with various concentrations of antibody 53E9, until equilibrium in bulk solution was established (Fig. 2). Subsequently, the concentrations of free FABP were estimated by short (10 min) measurements of the rate of precipitation on silica surfaces with small surface areas covered with a 53E9 catcher, and another, HRP-marked, antibody against FABP (118 ng/ml). It is important that the equilibrium in the liquid phase is not disturbed by the latter procedure. Hence, without washing (one-step process), free FABP concentrations were measured from the rate of precipitate formation after addition of DAB-H₂O₂ substrate. The dissociation constant K_{d} of the FABP-53E9 interaction determined in this way was 8 nM.

### Example 3:

### Determination of the dissociation constant for the IL6-αIL6.16 complex in blood plasma

High-affinity anti-human IL6 antibodies were a kind gift of Prof. L. Aarden, Sanquin Research, Amsterdam. Plasma (5x diluted) was incubated during 60 minutes with various concentrations of the high-affinity monoclonal anti-human IL6 antibody aIL6.16, as indicated in Figure 3. The medium was contacted for an additional 120 min with an αIL6.16-coated silicon slide. Subsequently, a one-step process according to the invention was performed to determine the free IL6 concentration. The value of the dissociation constant K_{d} deduced from the simulated curve (Figure 3) was 24 pM.

### Example 4:

### Determination of binding constants of α-IL6.16

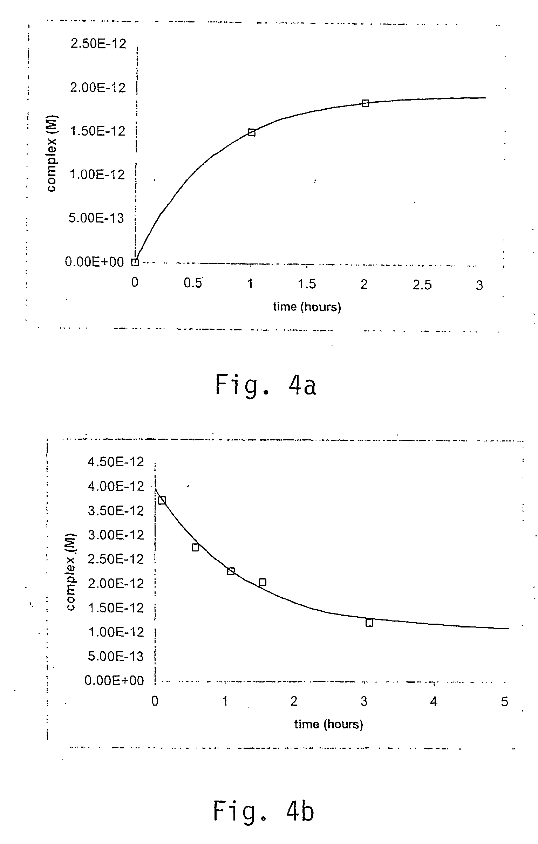

First IL6 (4 pM) was incubated with αIL6.16 (30 pM) for various time intervals, as indicated in Figure 4a. The medium was then incubated for an additional 10 minutes with an αIL6.16-coated silicon slide. Subsequently, the one-step process according to the invention was performed to determine the free IL6 concentration. K_{d}, kₒₙ and k_{off} as calculated from the simulated curve were 29 pM, 6.8 10⁶ M⁻¹s⁻¹ and 0,00020 s⁻¹, respectively.

Using a "reverse"process, IL6 (400 pM) was first preincubated with αIL6.16 (1 nM) for 50 minutes. The pre-incubation medium was then diluted 100-fold. After various time intervals, the medium was contacted for an addition 10 minutes with an αIL6.16-coated silicon slide. Subsequently, the one-step process according to the invention was carried out to determine the free IL6-concentration. K_{d}, kₒₙ and k_{off} from the simulated curve (Figure 4b) were 25 pM, 6.3 10⁶ M⁻¹s⁻¹ and 0,00016 s⁻¹, respectively.

### Example 5:

### Slides.

Several slides (5.1-5.4) were produced as follows.
5.1. Slides cut from phosphorus-doped and silica-covered silicon wafers were covered with PVC-layers with a thickness of 10-30 nm. This was done by retraction of the slide from a solution of PVC in cyclohexanone at controlled speeds, using a mechanical dipping device.
5.2. Slides cut from phosphorus-doped and silica-covered silicon wafers were covered with thin (1-5 nm) silane layers, using Sigmacote® and a protocol as indicated by the manufacturer.
5.3. Slides cut from phosphorus-doped and silica-covered silicon wafers were amino-terminated by immersion in a solution of (3-aminopropyl)trietoxysilane (Sigma) in acetone. Using the N-succinimidyl 3-(2-pyridyldithio)proprionate (SPDP) reagent, such slides were used for covalent binding of antibodies.
5.4. Streptavidin was covalently coupled to amino-terminated slides produced as described in 5.3. These slides were used for covalent binding of biotinylated antibodies.

### Example 6:

### Kits.

Kits (6.1. and 6.2.) were assembled as follows.
6.1. A kit comprising a suitable HRP-marked antibody was assembled as follows.
   Eight highly hydrophobic slides, prepared as described in Example 5 (5.1), together with HRP-marked 66E2 anti-human FABP (marker) a recombinant human FABP standard, blocking buffer, and precipitate forming HRP substrate (DAB+H₂0₂).
   To use this kit, the anti-FABP antibodies to be characterised should be coupled to the slides by overnight incubation in 2 mg/L of antibody at 4°C. The slides should then be incubated in blocking buffer. A known amount of FABP was preincubated for 30 minutes with five different concentrations of antibody and then added to ellipsometer cells, containing the slides. Using the one-step procedure according to the invention, including three calibrators, the amounts of free FABP can be measured. The kit will also contain two extra slides, to which FABP must be coupled, in order to check that the antibody to be characterised does not compete with the 66E2 anti-FABP tag.
6.2. A kit comprising a polyclonal anti-mouse IgG was assembled as follows.

Eight slides of aforementioned types 5.1-5.4 (Example 5), allowing either passive or covalent coupling of the antigen to the slide, HRP-marked anti-mouse IgG (marker), blocking buffer, and precipitating HRP substrate (DAB + H₂0₂).

To use this kit, the antigen used by the customer should be coupled to the slides and then incubated in blocking buffer. A fixed concentration of the antibody to be characterized should be pre-incubated with five different concentrations of antigen and then added to ellipsometer cells. Using the one-step procedure according to the invention, including three calibrators the amounts of free antibody can then be measured.

## Claims

1. A process for determining binding parameters, including dissociation constants and sorption rate constants, of the binding between a biomolecule and a binding partner thereof, both being present in a liquid phase, and comprising a marker-free binding step, **characterised by** the following steps:
(a) attaching either
(a1) the binding partner, or
(a2) the biomolecule, to a solid surface;
(b) allowing binding between the binding partner and the biomolecule, both in the liquid phase and on the solid surface;
(c) attaching a marker to:
(c1) the biomolecule, if said binding partner was attached to the solid surface according to step (a1), or to
(c2) the binding partner; if said biomolecule was attached to the solid surface according to step (a2);
(d) allowing the marker to produce a precipitate;
(e) detecting said precipitate as it is formed on said solid surface by determining a change of surface mass on the solid surface due to the formation of said precipitate;
(f) determining the free concentrations of the binding partner or the biomolecule in the liquid phase by comparing the rate of the formation of the precipitate with a standard curve, and
(g) calculate the binding constants from the free concentrations of the binding partner or the biomolecule thus determined.

2. The process according to claim 1, **characterised in that** the biomolecule is an enzyme and the binding partner is an inhibitor or a substrate therefore.

3. The process according to claim 1, **characterised in that** the biomolecule is a cell membrane protein and the binding partner is a ligand therefore.

4. The process according to claim 3, **characterised in that** the cell membrane protein is a receptor.

5. The process according to claim 1, **characterised in that** the biomolecule is a cell membrane protein and the binding partner is a cell membrane structure.

6. The process according to claim 5, **characterised in that** the cell membrane structure is artificial.

7. The process according to claim 5 or claim 6, **characterised in that** the cell membrane structure is a complete cell.

8. The process according to claim 1, **characterised in that** the biomolecule is an antibody or a fragment thereof, and the binding partner is an antigen of said antibody or fragment.

9. The process according to any one of claims 1 to 8, **characterised in that** the marker is an enzyme producing a precipitate from a soluble substrate.

10. The process according to any one of claims 1 to 9, **characterised in that** the solid surface is a silicon slide.

11. The process according to any one of claims 1 to 9, **characterised in that** the solid surface is a chromium-sputtered glass.

12. The process according to any one of claims 1 to 11, **characterised in that** the determination of the change of surface mass of step (e) is carried out by ellipsometry.

13. The process according to any one of claims 1 to 12, **characterised in that** the step of determining a change of surface mass is carried out on a surface area of less than 3.0 mm².

14. The process according to any one of claims 1 tot 13, **characterised in that** the dissociation constant is lower than 10⁻⁹ M.

15. The process according to claim 14, **characterised in that** the dissociation constant is 10⁻⁹ M - 10⁻¹³ M.

## Patentansprüche

1. Verfahren zur Bestimmung von Bindungsparametern, einschließlich Dissoziationskonstanten und Sorptionsgeschwindigkeitskonstanten, der Bindung zwischen einem Biomolekül und einem Bindungspartner dafür, die beide in einer flüssigen Phase vorliegen, das einen Bindungsschritt ohne Marker umfasst, bei dem man:
(a) entweder
(a1) den Bindungspartner oder
(a2) das Biomolekül
an einer festen Oberfläche anbringt,
(b) die Bindung zwischen dem Bindungspartner und dem Biomolekül sowohl in der flüssigen Phase als auch auf der festen Oberfläche zulässt,
(c) einen Marker an:
(c1) dem Biomolekül, wenn gemäß Schritt (a1) der Bindungspartner an der festen Oberfläche angebracht wurde, oder
(c2) dem Bindungspartner, wenn gemäß Schritt (a2) das Biomolekül an der festen Oberfläche angebracht wurde, anbringt,
(d) den Marker einen Niederschlag erzeugen lässt,
(e) den Niederschlag bestimmt, der sich auf der festen Oberfläche bildet, indem man eine Änderung der Masse der Oberfläche auf der festen Oberfläche in Folge der Bildung des Niederschlags bestimmt,
(f) die freien Konzentrationen der Bindungspartner oder des Biomoleküls in der flüssigen Phase bestimmt, indem man die Geschwindigkeit der Bildung des Niederschlags mit einer Kalibrierkurve vergleicht und
(g) die Bindungskonstanten aus den somit bestimmten freien Konzentrationen der Bindungspartner oder des Biomoleküls berechnet.

2. Verfahren nach Anspruch 1, **dadurch gekennzeichnet, dass** das Biomolekül ein Enzym ist und der Bindungspartner ein Inhibitor oder ein Substrat dafür ist.

3. Verfahren nach Anspruch 1, **dadurch gekennzeichnet, dass** das Biomolekül ein Zellmembranprotein und der Bindungspartner ein Ligand dafür ist.

4. Verfahren nach Anspruch 3, **dadurch gekennzeichnet, dass** das Zellmembranprotein ein Rezeptor ist.

5. Verfahren nach Anspruch 1, **dadurch gekennzeichnet, dass** das Biomolekül ein Zellmembranprotein und der Bindungspartner eine Zellmembranstruktur ist.

6. Verfahren nach Anspruch 5, **dadurch gekennzeichnet, dass** die Zellmembranstruktur künstlich ist.

7. Verfahren nach Anspruch 5 oder Anspruch 6, **dadurch gekennzeichnet, dass** die Zellmembranstruktur eine vollständige Zelle ist.

8. Verfahren nach Anspruch 1, **dadurch gekennzeichnet, dass** das Biomolekül ein Antikörper oder Fragment davon ist und der Bindungspartner ein Antigen des Antikörpers oder Fragments ist.

9. Verfahren nach einem der Ansprüche 1 bis 8, **dadurch gekennzeichnet, dass** der Marker ein Enzym ist, das einen Niederschlag aus einem löslichen Substrat erzeugt.

10. Verfahren nach einem der Ansprüche 1 bis 9, **dadurch gekennzeichnet, dass** die feste Oberfläche eine Siliciumfläche ist.

11. Verfahren nach einem der Ansprüche 1 bis 9, **dadurch gekennzeichnet, dass** die feste Oberfläche ein mit Chrom gesputtertes Glas ist.

12. Verfahren nach einem der Ansprüche 1 bis 11, **dadurch gekennzeichnet, dass** die Bestimmung der Änderung der Masse der Oberfläche gemäß Stufe (e) durch Ellipsometrie erfolgt.

13. Verfahren nach einem der Ansprüche 1 bis 12, **dadurch gekennzeichnet, dass** der Schritt der Bestimmung der Änderung der Masse der Oberfläche auf einem Oberflächenbereich von weniger als 3,0 mm² durchgeführt wird.

14. Verfahren nach einem der Ansprüche 1 bis 13, **dadurch gekennzeichnet, dass** die Dissoziationskonstante kleiner als 10⁻⁹ M ist.

15. Verfahren nach Anspruch 14, **dadurch gekennzeichnet, dass** die Dissoziationskonstante 10⁻⁹ M - 10⁻¹³ M beträgt.

## Revendications

1. Procédé de détermination de paramètres de liaison, comprenant des constantes de dissociation et des constantes de vitesse de sorption, de la liaison entre une biomolécule et son partenaire de liaison qui sont tous deux présents dans une phase liquide, et comprenant une étape de liaison exempte de marqueur, **caractérisé par** les étapes suivantes consistant à :
(a) attacher
(a1) soit le partenaire de liaison,
(a2) soit la biomolécule à une surface solide ;
(b) permettre la liaison entre le partenaire de liaison et la biomolécule, à la fois dans la phase liquide et sur la surface solide ;
(c) attacher un marqueur ;
(c1) à la biomolécule si ledit partenaire de liaison a été attaché à la surface solide selon l'étape (a1), ou
(c2) au partenaire de liaison si ladite biomolécule a été attachée à la surface solide selon l'étape (a2) ;
(d) permettre au marqueur de produire un précipité ;
(e) détecter ledit précipité à mesure qu'il se forme sur ladite surface solide en déterminant une modification de la masse surfacique sur la surface solide résultant de la formation dudit précipité ;
(f) déterminer les concentrations libres du partenaire de liaison ou de la biomolécule dans la phase liquide en comparant la vitesse de formation du précipité avec une courbe étalon, et
(g) calculer les constantes de liaison à partir des concentrations libres du partenaire de liaison ou de la biomolécule ainsi déterminées.

2. Procédé selon la revendication 1, **caractérisé en ce que** la biomolécule est une enzyme et le partenaire de liaison est un inhibiteur ou un substrat de cette enzyme.

3. Procédé selon la revendication 1, **caractérisé en ce que** la biomolécule est une protéine de la membrane cellulaire et le partenaire de liaison est un ligand de cette protéine.

4. Procédé selon la revendication 3, **caractérisé en ce que** la protéine de la membrane cellulaire est un récepteur.

5. Procédé selon la revendication 1, **caractérisé en ce que** la biomolécule est une protéine de la membrane cellulaire et le partenaire de liaison est une structure de la membrane cellulaire.

6. Procédé selon la revendication 5, **caractérisé en ce que** la structure de la membrane cellulaire est artificielle.

7. Procédé selon la revendication 5 ou la revendication 6, **caractérisé en ce que** la structure de la membrane cellulaire est une cellule complète.

8. Procédé selon la revendication 1, **caractérisé en ce que** la biomolécule est un anticorps ou un fragment d'anticorps et le partenaire de liaison est un antigène dudit anticorps ou fragment d'anticorps.

9. Procédé selon l'une quelconque des revendications 1 à 8, **caractérisé en ce que** le marqueur est une enzyme produisant un précipité à partir d'un substrat soluble.

10. Procédé selon l'une quelconque des revendications 1 à 9, **caractérisé en ce que** la surface solide est une lamelle en silicium.

11. Procédé selon l'une quelconque des revendications 1 à 9, **caractérisé en ce que** la surface solide est un verre revêtu de chrome par pulvérisation cathodique.

12. Procédé selon l'une quelconque des revendications 1 à 11, **caractérisé en ce que** la détermination de la modification de la masse surfacique de l'étape (e) se fait par ellipsométrie.

13. Procédé selon l'une quelconque des revendications 1 à 12, **caractérisé en ce que** l'étape de détermination d'une modification de la masse surfacique se fait sur une aire de surface inférieure à 3,0 mm².

14. Procédé selon l'une quelconque des revendications 1 à 13, **caractérisé en ce que** la constante de dissociation est inférieure à 10⁻⁹ M.

15. Procédé selon la revendication 14, **caractérisé en ce que** la constante de dissociation se situe dans la plage de 10⁻⁹ M à 10⁻¹³ M.
